Europäisches Patentamt
European Patent Office
Office européen des brevets

(19)

(11) Publication number: **0 352 817 A2**

(12) **EUROPEAN PATENT APPLICATION**

(21) Application number: 89114015.4

(22) Date of filing: 28.07.89

(51) Int. Cl.[4]: G01N 33/94 , C12P 21/00

(30) Priority: 29.07.88 JP 188323/88
04.08.88 JP 193357/88

(43) Date of publication of application:
31.01.90 Bulletin 90/05

(84) Designated Contracting States:
DE FR GB

(71) Applicant: ORIENTAL YEAST CO., LTD.
6-10, Azusawa 3-chome Itabashi-ku
Tokyo(JP)

(72) Inventor: Matsukawa, Hirokazu Nishi 1-1205, 6-ban
Sotojima-cho
Moriguchi-shi Oosaka-fu(JP)
Inventor: Takagahara, Isamu 5-3-59, Fushimidai
Inagawa-cho
Kawabe-gun Hyogo-ken(JP)
Inventor: Marui, Yoji
A-801, 4-ban Kosaka 3-chome
Higashioosaka-shi Oosaka-fu(JP)
Inventor: Tanaka, Hidetake
5-105-3, Midorigaoka
Itami-shi Hyogo-ken(JP)
Inventor: Hayashi, Chyozo
1-2-610, Takahata-cho
Nishinomiya-shi Hyogo-ken(JP)

(74) Representative: Thomsen, Dieter, Dr.rer.nat.
Kaiser-Ludwig-Platz 6
D-8000 München 2(DE)

# (54) Monoclonal antibody to catecholamine metabolite and quantitative assay for catecholamine metabolite.

(57) The present invention relates to a monoclonal antibody against a catecholamine metabolite and a process for production of said antibody as well as a method for quantitative assay for the catecholamine metabolite containing 3-methoxy, 4-hydroxy group using the monoclonal antibody to the catecholamine metabolite.

FIG. 1

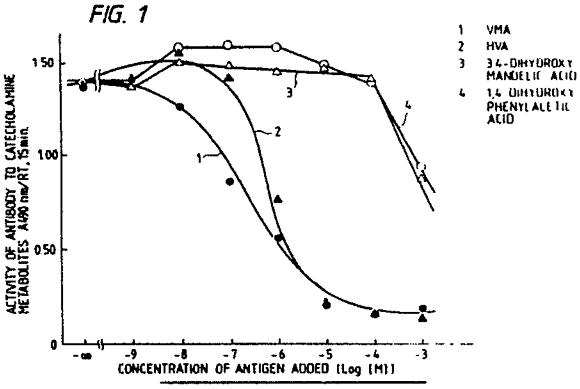


EP 0 352 817 A2

# MONOCLONAL ANTIBODY TO CATECHOLAMINE METABOLITE AND QUANTITATIVE ASSAY FOR CATECHOLAMINE METABOLITE

The present invention relates to monoclonal antibodies to catecholamine metabolites and a process for production thereof.

The present invention further relates to a method for quantitative assay for catecholamine metabolites containing 3-methoxy, 4-hydroxy group therein using the monoclonal antibodies to the catecholamine metabolites.

It is found that catecholamine metabolites generally excreted in human urine, inter alia, vanillylmandelic acid (hereafter simply referred to as VMA) and homovanillic acid (hereafter simply referred to as HVA), become markers for various diseases.

However, it has been recently clarified that both HVA and VMA, especially HVA, show a high level in neuroblastoma and tend to be significant as items for screening of neuroblastoma in newborn.

Monoclonal antibodies of the present invention to catecholamine metabolites take an important role for quantitative determination of VMA and HVA.

The method for determining catecholamine metabolite according to the present invention enables quantitative assay for HVA and VMA in an extremely simple way and thus greatly contributes to diagnosis of associated diseases.

Any method for quantitative determination of VMA and HVA in a simple and quick manner has not been known heretofore.

A conventional method comprises a step of separating analytes co-present with contaminants in urine (pretreatment) and a step of fractionation and determination of VMA and HVA extracted (analysis) but is complicated since the method involves complicated pretreatment, analysis using HPLC (high performance liquid chromatography), etc., including processing operations. The method also encounters various defects that the analysis is time consuming.

The problems in the prior art are summarized below.

1. Contaminants other than VMA and HVA should be removed by a treatment with organic solvents, or the like.

2. Depending upon measurement system, VMA and HVA must be converted into derivatives, respectively (gas chromatography) so that procedures for analysis are complicated.

3. Special devices are necessary (gas chromatography, HPLC method, GC-MS method) which render application to automated analysis system difficult.

4. Analytical devices or analytical procedures are special so that determination cannot be made by anyone in a rapid and simple way.

## SUMMARY OF THE INVENTION

The present inventors made extensive investigations under a concept that if VMA and HVA can be determined immunologically, VMA and HVA would be able to determine extremely easily and as a result, the inventors have succeeded in obtaining monoclonal antibodies which can be used for quantitative determination of VMA and HVA.

The present invention relates to monoclonal antibodies to catecholamine metabolites containing 3-methoxy, 4-hydroxy group therein.

Furthermore, the present inventors also made extensive investigations under a concept that if the system of capable of fractionation and determination of both VMA and HVA in a rapid and simple way can be established, early diagonosis or monitoring of neuroblastoma or metabolic abnormalities can be effected and as a result, the inventors have found that by using monoclonal antibodies capable of specifically reacting with catecholamine metabolites containing 3-methoxy, 4-hydroxy group therein, VMA and HVA can be quantitatively determined by fractionation assay through competitive enzyme immunoassay.

Thus, the present invention relates to a method for determination of catecholamine metabolites which comprises quantitative fractionation and determination of catecholamine metabolites containing 3-methoxy, 4-hydroxy group therein using monoclonal antibodies capable of specifically reacting therewith through competitive enzyme immunoassay.

Still further the present invention relates to a method for quantitative determination of catecholamine metabolites in competitive enzyme immunoassay characterized in that immunochemical reaction between antigens and antibodies is regulated by controlling environmental conditions to change affinity of monoclonal antibodies against various catecholamine metabolites and subjecting each of the catecholamine metabolites to quantitative fractionation and determination.

Still furthermore the present invention relates to a method for quantitative determination of catecholamine metabolites characterized in that the catecholamine metabolite containing 3-methoxy, 4-hydroxy group therein is either vanillylmandelic acid or homovanillic acid.

## BRIEF DESCRIPTION OF THE DRAWINGS

Fig. 1 shows reactivity of monoclonal antibody of the present invention against catecholamine metabolites containing 3-methoxy, 4-hydroxy group therein and their catechol derivertives in competitive enzyme immunoassay described in example 1.

Fig. 2 shows purification of said monoclonal antibody on protein A immobilized gel.

Fig. 3 shows the binding ability of monoclonal antibody to catecholamine metabolites, ℓ-VMA and HVA in pH range from 3.0 to 10.0 in example 2.

Fig. 4 shows standard curves for fractionation and determination of ℓ-VMA and HVA in competitive EIA system, wherein A indicates curve at pH 8.0 and B at pH 6.0.

## DETAILED DESCRIPTION OF THE PREFERRED EMBODIMENTS

Next, a process for producing monoclonal antibodies capable of specifically reacting catecholamine metabolites containing 3-methoxy, 4-hydroxy group therein is illustratively described below. Specific embodiments are later described in Example 1.

The monoclonal antibody of the present invention can be produced as follows. Catecholamine metabolites are immunized in BABL/c mice. After antibody titer to the immunized antigen is significantly noted in antisera by enzyme immunoassay (EIA). etc., spleen cells are removed and subjected to cell fusion with mouse myeloma cell line (hypoxanthine guanine phosphoribosyl transferase: HGRP-deficient strain, sensitive to HAT medium, XAg·8.6.5.3, etc.) to make immortalized cells capable of producing a monoclonal antibody to the antigen.

The catecholamine metabolites as an immune antigen includes a catecholamine metabolite containing 3-methoxy, 4-hydroxy group therein, for example, VMA, HVA, metanephrine, etc. Any of them may be usable.

The catecholamine metabolites containing 3-methoxy, 4-hydroxy group, for example, commercially available d,ℓ-VMA can be bound to a carrier protein, for example, KLH, BSA, etc. in the presence of a crosslinking reagent to produce antigens.

The resulting d,ℓ-VMA antigen is immunized in the intraperitoneal cavity of BALB/c mice together with Freund's complete adjuvant or incomplete adjuvant. After immunization, blood is in part collected and an antibody titer to d,ℓ-VMA in antisera is determined by immunoassays, such as passive agglutination, EIA and RIA, preferably by EIA.

In the case of determination by EIA, an antibody titer, for example, to immobilized VMA antigen can be determined by using horse radish peroxidase-labeled rabbit anti-mouse IgG antibody as secondary antibody, assaying a quantity of mouse immunoglobulin bound to the immobilized VMA antigen present in antisera.

If a reasonable increase in antibody titer is noted, VMA immunogen is boosted, then spleen cells are collected from the immunized animals, from which mouse hybridoma cells can be obtained by cell fusion with mouse myeloma cell line (XAg 8.6.5.3) which was made immortal and using polyethylene glycol (M.W. 1,500) as a fusing agent. Fused cells alone are proliferated in HAT selection medium and activity of antibody specific to VMA antigen present in the culture supernatant is detected by EIA described above, whereby the monoclonal antibody of the present invention can be produced.

If the determination of the catecholamine metabolite according to the present invention, the monoclonal antibody to catecholamine metabolite is used.

In more detail, the present invention provides a method for quantitative assay of the catecholamine metabolite containing 3-methoxy, 4-hydroxy group therein by competitive enzyme immunoassay, using the monoclonal antibody produced by the process described above which specifically reacts with the catecholamine metabolite containing 3-methoxy, 4-hydroxy group therein.

As the result of detailed study of immunochemical reactions of monoclonal antibodies and catecholamine derivatives having 3-methoxy, 4-hydroxy group as antigens, especially in VMA and HVA, it has been found that affinity of the antibodies to them are varied depending upon environmental conditions (factors) where immunochemical rection is carried out and affinity ratios of the antibodies against both antigens differ from each other; based on the finding, the fractionation and quantitative determination has been made possible.

It is generally art-recognized that immunochemical reaction between antigen and antibody is effected by interactions such as:

1. ionic bond
2. hydrophobic bond
3. hydrogen bond
4. van der Waals force etc. in combination. These bonds are not formed covalently so that the bonds are cleaved by reasonable environmental changes, whereby the once formed immune complexes are broken and the antigen and antibody are reverted to their free forms. Such a fact is widely known and its application is shown as immunoaffinity chromatography using antigen or antibody as immobilized ligand.

According to the present invention, the binding ability between the catecholamine metabolite containing 3-methoxy, 4-hydroxy group therein and the monoclonal antibody to the metabolite and interaction therebetween are affected by dimethylsulfoxide and ethylene glycol which give influence on hydrogen bond, a concentration of salts such as NaCl which is considered to weaken the ionic bond and furthermore more strongly by pH. Therefore, a standard curve showing binding ability of the monoclonal antibody to each substance in various environmental conditions is previously determined and a test sample such as urine, etc. is measured under the same environmental conditions and thus, a content of the substance and a content ratio can be quantitatively determined quickly.

Next, the present invention is described by referring to examples.

Example 1

Preparation of monoclonal antibody capable of specifically reacting with catecholamine metabolites containing 3-methoxy, 4-hydroxy group therein

(1) Immunization in BALB/c mice and cell fusion:

Commercially available d,ℓ-vanillymandelic acid (VMA) was previously bound to kyholelimpet hemocyanin (KLH) using water-soluble carbodiimide (EDC) and 50 µg of the resulting VMA-KHL complex was intraperitoneally injected to BALB/c mice (male at the age of 4 weeks) together with Freund's complete adjuvant. Then, supplemental immunization was performed twice every other week with the same dose of the VMA-KLH complex together with Freund's incomplete adjuvant. Three or four days before cell fusion with mouse myeloma cells (XAg 8.6.5.3), a solution of 10 µg of the VMA-KLH complex in PBS (-) was intraperitoneally boosted.

The obtained spleen cells and mouse myeloma cells were subjected to cell fusion in a conventional manner using 50% (w/v) polyethylene glycol (M.W., 1,500). The fused cells were inoculated on a 48 well microplate (Coaster Inc.) in $10^6$ cells per well. Then, hybridomas alone were selectively cultured in 15% FCS-containing HAT medium (RPMI-1640 medium containing hypoxanthine, aminoputerine and thymidine).

(2) Screening of monoclonal antibody specific to the catecholamine metabolite and its characteristic reactivity:

Microtiter plate (Nunc Inc.) for EIA to which VMA-BSA complex had been physicochemically adsorbed was used. The supernatant obtained by culturing the hybridoma was added to the microtiter plate and reacted for screening of the monoclonal antibody specific to the catecholamine metabolite containing 3-methoxy, 4-hydroxy group therein.

Actual measurement was performed as follows: 100 µl of the hybridoma culture supernatant was added to the VMA-BSA-immobilized microtiter plate described above followed by incubation at 37 °C for 60 minutes. After thoroughly washing, the adsorbed mouse immunoglobulin was reacted with HRPOD (horse radish peroxidase)-labeled rabbit anti-mouse IgG (H & L chain) antibody (Miles Corp.) under similar conditions. 0.1 M Potassium phosphate buffer (pH 6.0) containing 100 μl of 10 mg/ml of o-phenylenediamine and 0.01% (v/v) $H_2O_2$ was added as an enzyme reaction solution. After the reaction was stopped by adding 4 N HCl, optical density was measured at 490 nm.

The monoclonal antibody selected by the screening described above was examined with respect to catecholamine metabolites corresponding to catechol derivatives. The results are shown in Fig. 1. Measurement was performed by competitive EIA, under reaction conditions similar to the above-described screening, by adding catecholamine metabolites and their catechols at various concentrations. In the figure, the ordinate indicates an apparent reactivity of the antibody with catecholamine metabolites, wherein $IC_{50}$ value (a concentration of antigen necessary for 50% inhibition in the reaction system of each antigen is as shown below.

| | |
|---|---|
| Vanillylmandelic acid | 2.8 x $10^{-7}$ M |
| Homovanillylmandelic acid | 6.8 x $10^{-7}$ M |
| 3,4-Dihydroxymandelic acid | 1.4 x $10^{-3}$ M |
| 3,4-Dihydroxyphenylacetic acid | 1.4 x $10^{-3}$ M |

(3) Production of monoclonal antibody specific to catecholamine metabolites:

Hybridoma cell line (5 x $10^6$) capable of producing and secreting a monoclonal antibody specific to catecholamine metabolites was intraperitoneally administered to BALB/c mice (male, older than 4 weeks), to which 0.5 ml of pristane had been administered at a dose of 0.5 ml/mouse one week before. Ten to fourteen days after, the ascites was collected and the monoclonal antibody contained in the ascites was purified by affinity chromatography using protein A-immobilized Sepharose 4B gel. The results are shown in Fig. 2. In more detail, 1.5 M glycine buffer (pH 9.0) containing the same amount of 3 M NaCl as that of the ascites was throughly mixed with the ascites and the mixture was charged on protein A gel column equilibrated with the same buffer. After the non-adsorbed fraction was removed, the column was throughly washed with PBS(-). The monoclonal antibody adsorbed to the column was eluted with 0.1 M acetate buffer (pH 3.0).

The thus obtained monoclonal antibody is not reactive at all with catecholamine derivatives having 3,4-dihydroxy group. Furthermore, the monoclonal antibody is particularly well reactive with vanillylmandelic acid and homovanillic acid.

Example 2

(1) Coupling of d,ℓ-VMA to carrier protein (BSA):

In 0.5 ml of 0.01 N acetic acid, 5 mg of d,ℓ-VMA was dissolved together with 15 mg of water-soluble carbodiimide (WSC). Under ice cooling the mixture was settled for 15 minutes to activate α-carboxyl on the side chain of d,ℓ-VMA. Then, 0.5 ml of 0.1 M $NaHCO_3$ (pH 9.0) in which 10 mg of BSA had been dissolved was added to the system followed by coupling at 30°C for 90 minutes. After completion of the reaction, d,ℓ-VMA remained in an excess amount and water-soluble carbodiimide were removed by dialysis to PBS(-).

(2) Binding ability of immobilized d,ℓ-VMA to the monoclonal antibody specific against catecholamine metabolites containing 3-methoxy, 4-hydroxy group therein:

VMA-BSA (2 μg/ml) prepared in Example (1) was reacted with activated microtiter plate (96 well) manufactured by MMC Corp. Incubation was performed at 25°C overnight to immobilize VMA-BSA to the plate covalently. Then, VMA-SBA adsorbed onto the solid phase by the action other than the covalent bond was washed with 0.1 N acetic acid containing 0.1% (w/v) SDS. Thereafter, as shown in Fig. 3, the monoclonal antibody obtained in the preparation example which was dissolved at each pH value was added and, at the same time, ℓ-VMA and HVA, which are catecholamine metabolites containing 3-methoxy, 4-hydroxy group therein, were added to examine affinity ratio of the monoclonal antibody to the catecholamine metabolites under each environment (pH).

As is apparent from the results shown in Fig. 3, it is found that the reaction of the immobilized VMA-BSA and the monoclonal antibody is stable in a pH range of from 6.0 to 8.0. Furthermore, affinity ratio of monoclonal antibody to ℓ-VMA and HVA which are 2 kinds of catecholamine metabolites containing 3-methoxy, 4-hydroxy group therein is expressed in terms of a ratio of inhibitory degree in the two metabolites in EIA used. The results are as follows.

| | ℓ-VMA/HVA |
|---|---|
| pH 6.0 | 0.38 |
| pH 8.0 | 0.87 |

Accordingly, it is observed that the affinity to ℓ-VMA and to HVA changes at pH 6.0 and pH 8.0.

(3) Preparation of standard curve for fractionation and determination of ℓ-VMA and HVA in competitive EIA:

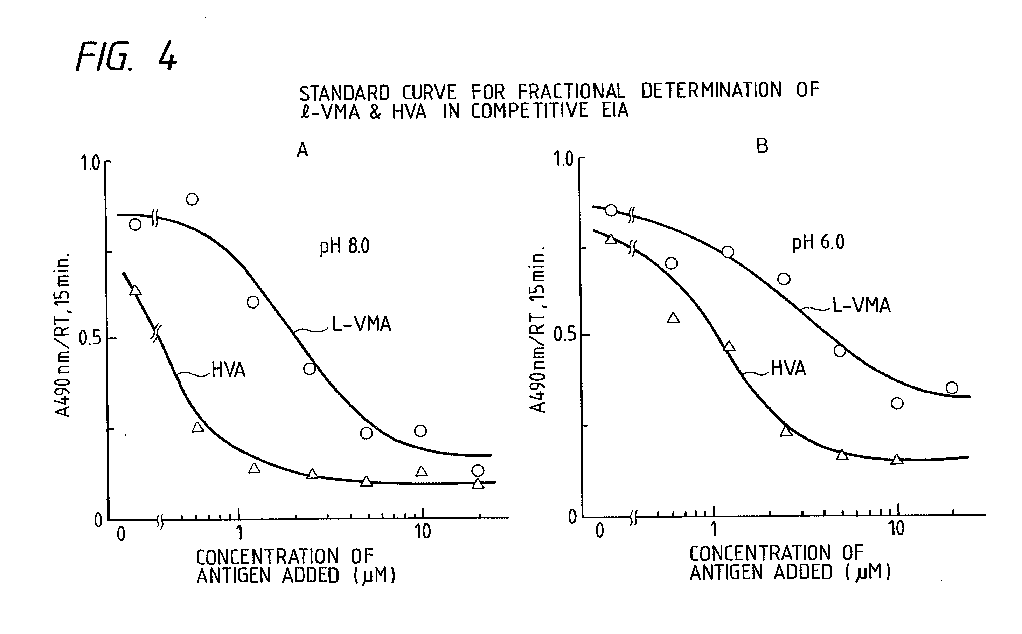

Based on the opertion in Example (2), immunochemical reaction was carried out under conditions at pH 6.0 and pH 8.0 to prepare respective standard curves for ℓ-VMA and HVA. The amount of the monoclonal antibody finally bound to the VMA-BSA solid phase was measured using HRPOD-labeled rabbit anti-mouse IgG antibody in Example (2) using HRPOD enzyme activity as its index. The results are shown in Fig. 4. At pH 8.0, the added antigen did not react with ℓ-VMA in an amount of less than 1 µM but reacted only with VMA; at pH 6.0, the antigen reacted with both ℓ-VMA and HVA in an amount of 1 µM to 10 µM.

Based on the foregoing results, the catecholamine metabolites contained in the test sample can be simultaneously determined under conditions of pH 8.0 and pH 6.0 and, ℓ-VMA and VMA can be calculated simultaneously from respective palindromic curves.

## Claims

1. A monoclonal antibody capable of specifically reacting with a catecholamine metabolite containing 3-methoxy, 4-hydroxy group therein.

2. A monoclonal antibody according to claim 1, which does not react with said catecholamine metabolite containing 3-methoxy, 4-hydroxy group and its metabolite at all.

3. A monoclonal antibody according to claim 1, which reacts particularly with vanillylmandelic acid and homovanillic acid among catecholamine metabolites.

4. A process for producing a monoclonal antibody according to claim 1, which comprises culturing a specific hybridoma cell line capable of producing a monoclonal antibody according to claim 1 in mouse ascites or serum-added or serum free medium.

5. A method for determination of a catecholamine metabolite which comprises fractionally determining a catecholamine metabolite containing 3-methoxy, 4-hydroxy group by competitive enzymeimmunoassay using a monoclonal antibody capable of specifically reacting with a catecholamine metabolite containing 3-methoxy, 4-hydroxy group.

6. A method for determination of a catecholamine metabolite by competitive enzymeimmunoassay according to claim 5, characterized by regulating an immunochemical reaction between an antigen and an antibody by controlling environmental conditions to change an affinity of said monoclonal antibody to various catecholamine metabolites and fractionally determining each of said catecholamine metabolites.

7. A method for determination of a catecholamine metabolite according to claim 5 or 6, wherein said catecholamine metabolite containing 3-methoxy, 4-hydroxy group is vanillylmandelic acid or homovanillic acid.

FIG. 1

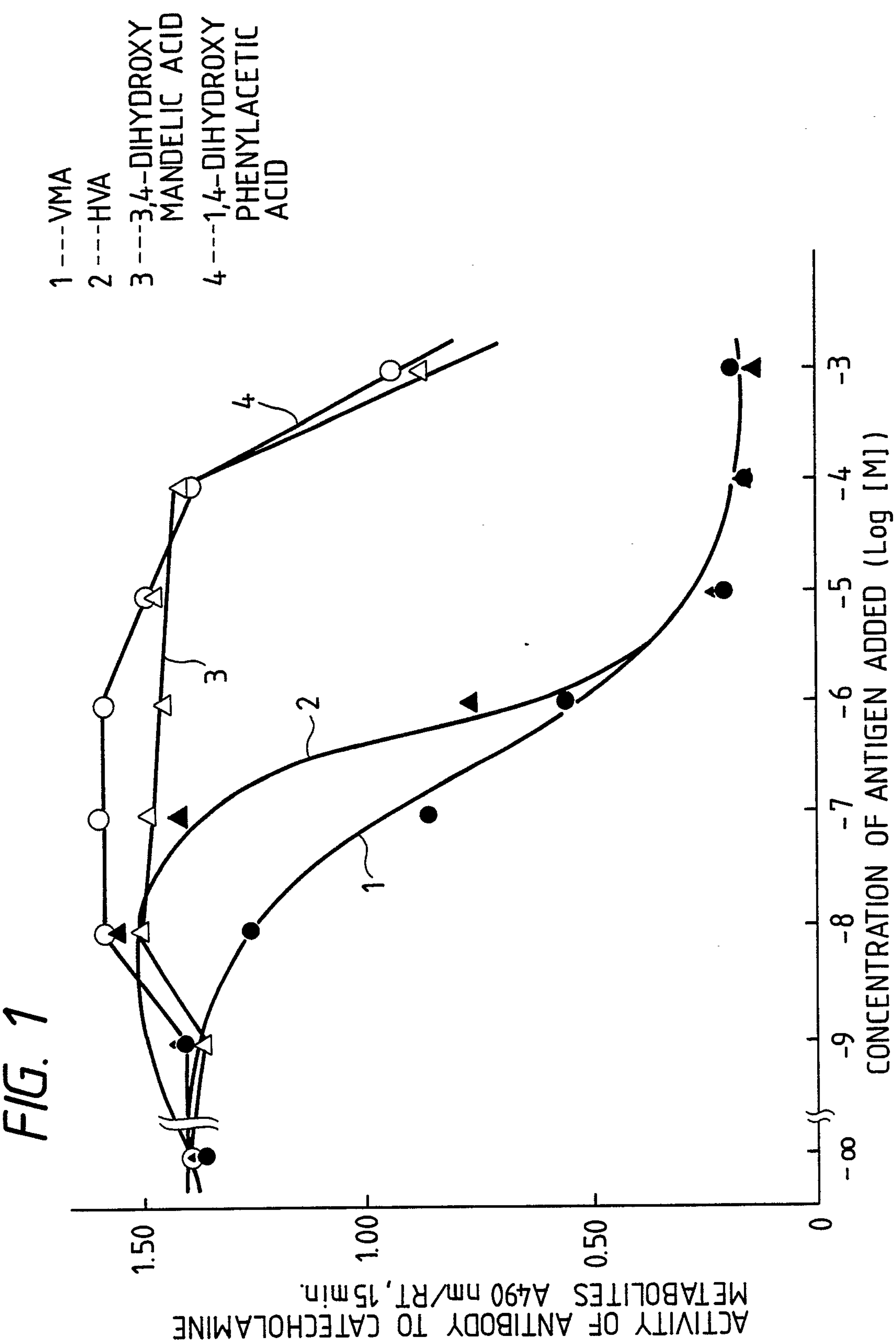
1 ---VMA
2 ---HVA
3 ---3,4-DIHYDROXY MANDELIC ACID
4 ---1,4-DIHYDROXY PHENYLACETIC ACID
ACTIVITY OF ANTIBODY TO CATECHOLAMINE METABOLITES A490 nm/RT, 15 min.
1.50
1.00
0.50
0
-∞
-9
-8
-7
-6
-5
-4
-3
CONCENTRATION OF ANTIGEN ADDED (Log [M])
1
2
3
4

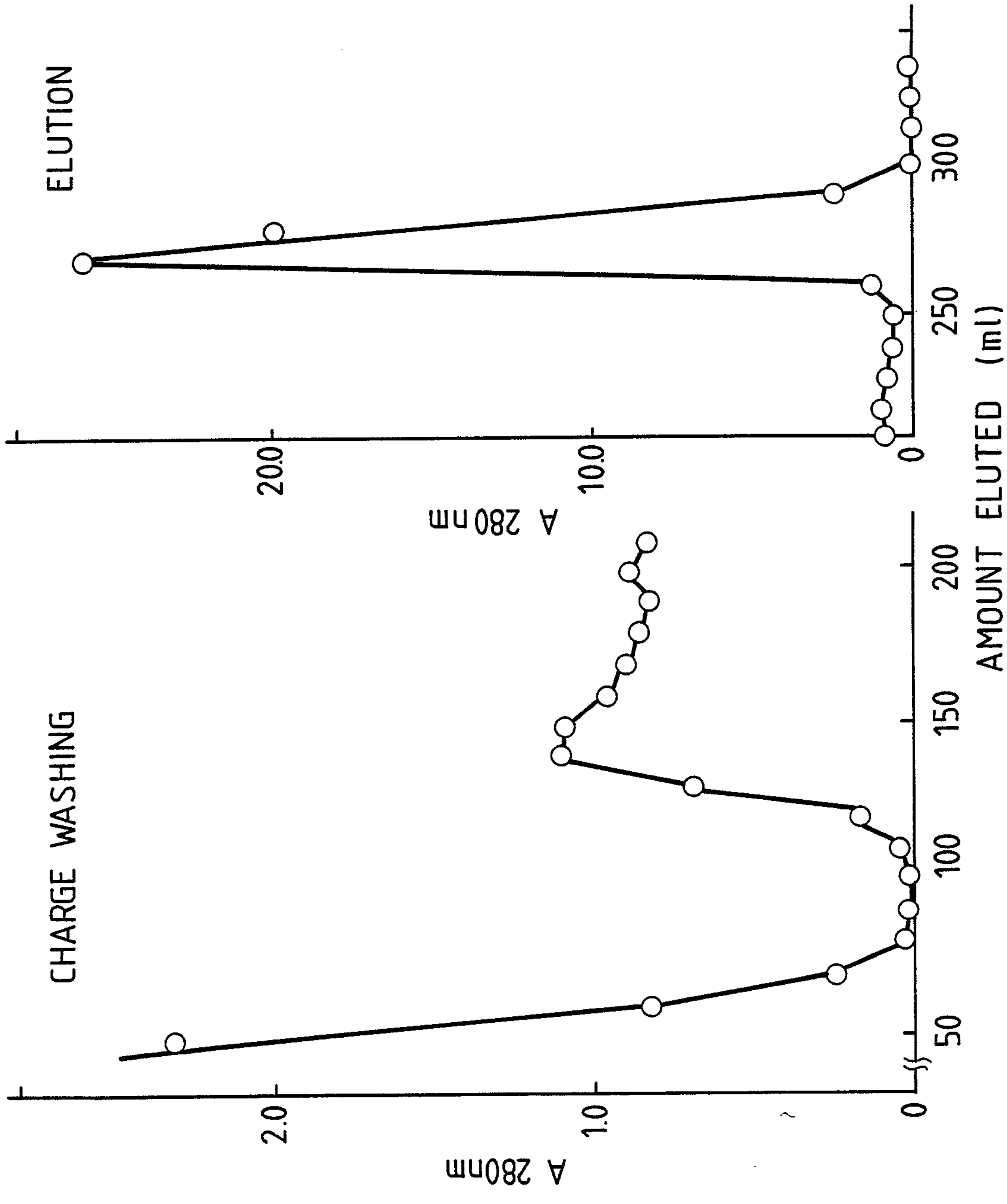
CHARGE WASHING
ELUTION
A 280nm
2.0
1.0
0
A 280nm
20.0
10.0
0
50
100
150
200
250
300
AMOUNT ELUTED (ml)

FIG. 2

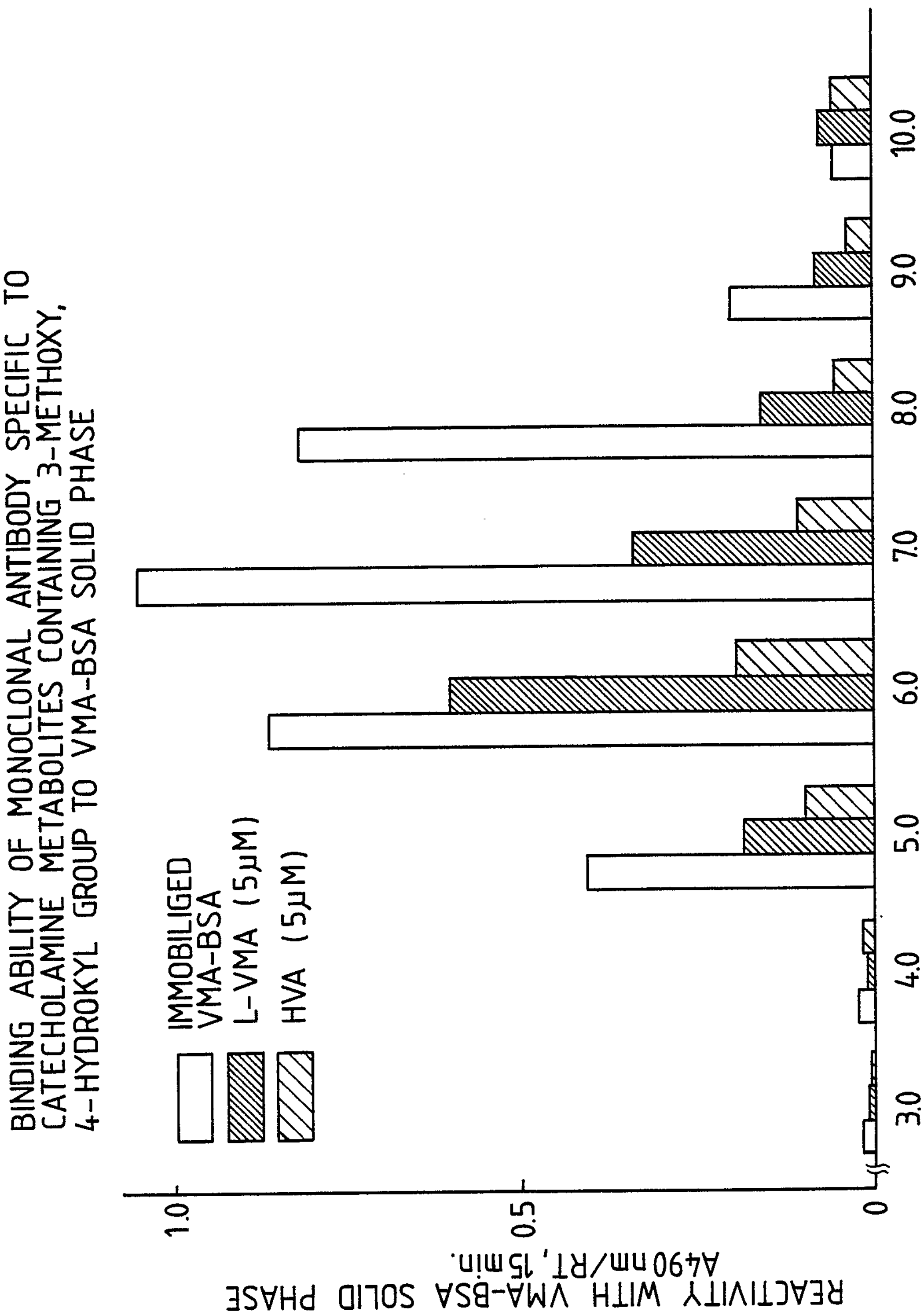
BINDING ABILITY OF MONOCLONAL ANTIBODY SPECIFIC TO CATECHOLAMINE METABOLITES CONTAINING 3-METHOXY, 4-HYDROKYL GROUP TO VMA-BSA SOLID PHASE
IMMOBILIGED VMA-BSA
L-VMA (5μM)
HVA (5μM)
REACTIVITY WITH VMA-BSA SOLID PHASE
A490 nm/RT, 15 min.
1.0
0.5
0
3.0
4.0
5.0
6.0
7.0
8.0
9.0
10.0
pH

FIG. 3

FIG. 4

STANDARD CURVE FOR FRACTIONAL DETERMINATION OF
ℓ-VMA & HVA IN COMPETITIVE EIA